(19) Europäisches Patentamt
European Patent Office
Office européen des brevets

(11) Veröffentlichungsnummer: **0 280 155 B1**

(12) **EUROPÄISCHE PATENTSCHRIFT**

(45) Veröffentlichungstag der Patentschrift: **20.05.92**

(51) Int. Cl.[5]: **B01J 13/02**, C12N 11/04, A61K 9/50

(21) Anmeldenummer: **88102228.9**

(22) Anmeldetag: **16.02.88**

# (54) Mikroenkapsulierung von biologisch aktivem Material.

(30) Priorität: **25.02.87 DE 3706010**

(43) Veröffentlichungstag der Anmeldung:
**31.08.88 Patentblatt 88/35**

(45) Bekanntmachung des Hinweises auf die Patenterteilung:
**20.05.92 Patentblatt 92/21**

(84) Benannte Vertragsstaaten:
**AT BE CH DE FR GB IT LI NL SE**

(56) Entgegenhaltungen:
**EP-A- 0 152 898**
**GB-A- 2 135 954**
**US-A- 3 594 327**

(73) Patentinhaber: **HOECHST AKTIENGESELLSCHAFT**
**Postfach 80 03 20**
**W-6230 Frankturt am Main 80(DE)**

(72) Erfinder: **Bader, Hubert, Dr.**
**Im Münchfeld 23**
**W-6500 Mainz(DE)**
Erfinder: **Keil, Karl-Heinz, Dr.**
**Lübecker Weg 3**
**W-6450 Hanau 6(DE)**
Erfinder: **Rüppel, Diether, Dr.**
**Karl-König-Weg 1**
**W-6230 Frankturt am Main 80(DE)**
Erfinder: **Schlingmann, Merten, Prof. Dr.**
**Schneidhainer Strasse 32a**
**W-6240 Königstein/Taunus(DE)**
Erfinder: **Walch, Axel, Dr.**
**Hans-Sachs-Strasse 5**
**W-6000 Frankturt am Main 90(DE)**

EP 0 280 155 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.08/2.19/2.0)

**Beschreibung**

Die Immobilisierung von Enzymen bzw. lebendem Zellmaterial ist weithin bekannt. Durch den Einschluß von aktivem Zellmaterial in Mikrokapseln, wie es z.B. in "Artificial Cells" T.M.S. Chang und C.C. Thomas Publ., Springfield Illinois (1972) beschrieben ist, läßt sich durch die Kompartimentierung unter Beibehaltung einer möglichst großen Oberfläche die biologische Aktivität des Materials erhalten bzw. verbessern.

Es sind eine Vielzahl von Verfahren zur Einkapselung von Zellen und Zellmaterial beschrieben, die meist darauf beruhen, daß das Zellmaterial in einer semipermeablen, wasserunlöslichen, biokompatiblen Membran eingeschlossen wird, die durch Reaktion von gelösten polyanionischen mit polykationischen Polymeren gebildet wird.

In der deutschen Offenlegungsschrift 30 12 233 wird ein Verfahren zum Einkapseln von Gewebe oder Einzelzellen beschrieben. Das Zellmaterial soll lebensfähig in geschütztem Zustand in einer Membran eingeschlossen sein, die zur Erhaltung der normalen Stoffwechselfunktionen der Zellen für Nährstoffe, Ionen, Sauerstoff und andere niedermolekulare Stoffe durchlässig ist. Das einzukapselnde Material wird in einem Medium suspendiert, das einen wasserlöslichen, in Tröpfchen gelierbaren Stoff enthält, um für das Gewebe eine vorübergehende schützende Umhüllung zu schaffen. Aufgrund der Empfindlichkeit des Zellmaterials können für die reversible Gelierung über ein Elektrolytmilieu nur sehr spezielle Puffermedien eingesetzt werden. Bevorzugte Stoffe für die Bildung der temporären Kapseln sind natürliche Polysaccharid-Gummiharze, die a) bei einer Änderung der Bedingungen, etwa des pH-Werts oder bei Zugabe von mehrwertigen Kationen, wie $Ca^{2+}$, zur reversiblen Bildung einer formhaltigen Masse befähigt sind und die b) mit Polybasen, deren Amingruppen mit sauren Polysaccharid-Bestandteilen reagieren können, dauerhaft komplexierbar sind. Nach Bildung der dauerhaften semipermeablen Membran kann die temporäre Kapsel durch Herstellung der Bedingungen, unter denen der Stoff flüssig ist, aufgelöst werden. Dies ist insbesondere erforderlich, wenn es sich um äußerst empfindliches, biologisch aktives Material handelt, das durch die zahlreichen, quervernetzten Gruppen des Polysaccharid-Harzes im Zellwachstum bzw. im Stoffwechsel behindert wird.

In der deutschen Offenlegungsschrift 32 09 127 wird ein Verfahren beschrieben, in dem Zellen nach der obengenannten Methode eingekapselt werden, um Stoffwechselprodukte zu gewinnen.

Biologisch aktives Material kann auch in Proteine mit weitgehend neutraler Nettoladung eingeschlossen werden, wie in der Europäischen Anmeldung 0 129 619 erwähnt ist. Dabei treten jedoch Probleme auf, die z.B. durch leichtere mikrobiologische Kontamination oder geringere Reproduzierbarkeit der Proteine hervorgerufen werden, aber vor allem durch die Tatsache, daß ein reversibler Gel-/Sol-Übergang nicht möglich ist.

In der europäischen Patentanmeldung 0 188 309 wird ein Verfahren beschrieben, bei dem lebendes Zellmaterial in eine biokompatible, semipermeable, wasserunlösliche Membran eingeschlossen wird, die durch Reaktion von polyanionischen und polykationischen Polymeren auf Acrylbasis gebildet wird. Dabei wird das polyanionische oder polykationische Acrylpolymer in Wasser gelöst und das Zellmaterial darin suspendiert. Die Suspension wird in Tropfenform in eine Lösung, die die elektrisch entgegengesetzt geladenen polykationischen bzw. polyanionischen Acrylpolymere enthält, gebracht, wobei sich an der Phasengrenze die Polymer-Polymer-Komplexmembran ausbildet.

In der europäischen Patentanmeldung 0 152 898 wird ein Verfahren beschrieben, bei dem ein aktives Material (Zellen, Mikroorganismen, Enzyme, Hormone, Antikörper, Katalysatoren oder Substrate) in eine Mikrokapsel eingeschlossen wird, deren Membran durch Reaktion eines anionischen oder kationischen Polymers mit einem ionischen Polymer entgegengegensetzter Ladung gebildet wird. Als einsetzbare anionische Polymere werden Alginat, Carragean, Hyaluronsäure, Carboxymethylcellulose, Xanthan, Furcellaran und sulfonierte organische Polymere offenbart; als kationische Chitosan, Polylysin, Polyethylamin und Polyvinylamin.

In der U.S.-Patentschrift 3 594 327 wird ein Verfahren zur Enkapsulierung von wasserunmischbaren Substanzen wie Eisenoxid beschrieben. Es wird erwähnt, daß das Verfahren geeignet ist Materialien einzukapseln, die pH-und/oder temperatursensitiv sind. In diesem Verfahren wird im letzten Verfahrensschritt die Zugabe von Kupfer- und Chromsalzen genannt, wodurch die gebildete Membran wasserunlöslich und stabil wird.

Das Ergebnis dieses Verfahrens sind Polyelektrolytmembrankapseln, bestehend aus einer semipermeablen Membran, welche aus einem Kupfer- oder Chrom-haltigen Polymerkomplex gebildet wird und einem von dieser eingeschlossenen Material.

Alle bisher beschriebenen Polyelektrolytmembrankapseln oder Verfahren zur Herstellung derselben geben jedoch keinerlei Hinweis darauf, welche Polyelektrolyte, insbesondere welche Polybasen sich in welchen Konzentrationen besonders gut eignen, um die folgenden Anforderungen an Polyelektrolytmembrankapseln zu erfüllen.

Zum einem müssen die eingesetzten Polyelektrolytkomplexe selbst und die um den Kern gebildete Membran stabil sein; zum anderen müssen die verwendeten Polyelektrolyte den biologischen und verfahrenstechnischen Anforderungen genügen.

Zu den biologischen Anforderungen zählen z.B.:
die Zellverträglichkeit, die Stabilität der Membran bei gegebener Ionenstärke oder die Reaktionstemperatur der verwendeten Polyelektrolyte.

Zu den verfahrenstechnischen Anforderungen zählen z.B.:
die Viskosität der Lösung. So soll die Lösung sich sowohl verdüsen lassen als auch Tropfen mit ausreichender Elastizität bilden.

Die Stabilität von Polyelektrolytmembranen hängt von einer Vielzahl von Parametern ab, z.B.:
von der Ladungsdichte, der Ladung, dem Molekulargewicht oder der Struktur (Konformation) der Polyelektrolyte.

Diese Parameter lassen sich zwar im Nachhinein ermitteln, jedoch läßt sich aus den idealisierten Einzelwerten nicht ohne weiteres das ideale Polymere, welches die oben gestellten Anforderungen erfüllt, ableiten, da diese Parameter meist erst in Kombination zu den gewünschten Eigenschaften führen.

Überraschenderweise wurden nun Polybasen zur Herstellung von Polyelektrolytmembrankapseln gefunden, die allen oben genannten Anforderungen genügen.

Die Erfindung betrifft daher:
Polyelektrolytmembrankapseln, bestehend aus einer semipermeablen Membran und einem von ihr eingeschlossenen biologisch aktiven Material, wobei die Membran aus einer biokompatiblen, nicht toxischen Polysäure und einer Polybase besteht, dadurch gekennzeichnet, daß die Polybase aus einem Polymer, gebildet aus wiederkehrenden Monomereinheiten der Formel (I)

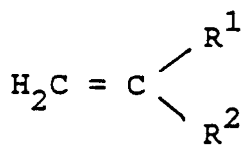

(I)

wobei
- $R^1$ Wasserstoff oder Methyl und
- $R^2$ eine Aminomethylgruppe, ein Imidazol-Rest oder ein Rest der Formel (II) ist

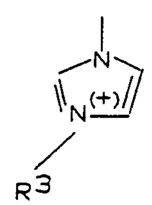

(II)

wobei
- $R^3$ Wasserstoff, Methyl oder Ethyl bedeutet, wobei miteinander verknüpfte Monomereinheiten auch voneinander verschiedene Reste $R^1$ und/oder $R^2$ enthalten können,
  und
  gegebenenfalls noch weiteren hydrophilen, biokompatiblen, keine elektrische Ladung aufweisenden Monomereinheiten

und
gegebenenfalls noch weiteren wasserlöslichen, biokompatiblen Polymeren, die gegebenenfalls mit dem Polymer, gebildet aus Monomereinheiten der Formel I und gegebenenfalls weiteren hydrophilen, biokompatiblen, keine elektrische Ladung aufweisenden Monomereinheiten, über überbrückende Einheiten vernetzt sind, besteht.

Weiterhin betrifft die Erfindung ein Verfahren zur Herstellung der erfindungsgemäßen Polyelektrolytmembrankapseln, deren nachträgliche Modifikation, z.B. Vernetzung sowie deren Verwendung zur Herstellung von Produkten durch Zellkulturen.

Im folgenden wird die Erfindung detailliert beschrieben.

Unter biologisch aktivem Material sind beispielsweise Zellen, Zellmaterial, Mikroorganismen, Enzyme, Hormone oder auch nicht biochemische Substanzen wie Substrate oder Katalysatoren zu verstehen.

Die Enkapsulierung des biologisch aktiven Materials geschieht in an sich bekannter Weise, wie es z.B. in den europäischen Offenlegungsschriften 0 152 898 oder 0 188 309 beschrieben ist. Dazu wird das biologisch aktive Material in einer 0,1-10 %igen wäßrigen Lösung der Polysäure gelöst oder suspendiert, in Tropfenform überführt und dann in eine 0,1-10 %ige wäßrige Lösung der Polybase gebracht, wobei sich an der Phasengrenze zwischen Polysäure und Polybase die semipermeable Membran ausbildet, die das biologisch aktiv Material einschließt. Durch Einsatz der obengenannten Polybasen lassen sich so Mikrokapseln mit einem Durchmesser von 100 bis 3000 µm herstellen. Die Tropfengröße läßt sich in bekannter Weise, z.B. über die Beschaffenheit der Düse einstellen. Die Düse besteht z.B. aus einer Kanüle (Innendurchmesser 0,1-1 mm). Diese kann konzentrisch in einen Hohlzylinder eingelassen sein, so daß über den entstehenden Ringspalt ein Gasstrom erzeugt werden kann, der die aus der Düse austretenden Tropfen tangential umströmt und abdrückt. Die Tropfengröße nimmt mit steigender Strömungsgeschwindigkeit des Gases ab.

Die verwendeten Polysäuren, die das biologisch aktive Material enthalten, sollten - speziell bei der Enkapsulierung von empfindlichem biologischem Material - bioverträglich sein. Zur Anwendung kommen hier beispielsweise Polysaccharide wie Alginat, Carragean, Carboxymethylcellulose oder Xanthan.

Die zur Herstellung der erfindungsgemäßen Polyelektrolytmembrankapseln verwendeten Polybasen gemäß Formel (I) sind, sofern nicht käuflich, in einfacher Weise herstellbar.

Durch Lösungspolymerisation eines oder gegebenenfalls auch mehrerer verschiedener Monomere gemäß Formel (I) und gegebenenfalls noch weiteren hydrophilen, biokompatiblen, elektrisch neutralen Monomeren, wie z.B. N-Vinylpyrrolidon, N-Vinylmethylacetamid, Vinylcaprolactam, Acrylsäure oder Acrylamide, erhält man eine Polybase, die entweder direkt eingesetzt werden kann oder gegebenenfalls noch mit weiteren wasserlöslichen, biokompatiblen Polymeren gemischt werden kann, wobei diese zusätzlich hinzugefügten Polymere auch noch mit den Polymeren, aufgebaut aus Monomereinheiten gemäß Formel (I), unter Hinzugabe von Epichlorhydrin vernetzt werden können. Als zusätzliche wasserlösliche, biokompatible Polymere können beispielsweise Celluloseether oder Kondensationsprodukte aus Dicarbonsäuren und Diaminen eingesetzt werden.

Bevorzugt bestehen die Polybasen aus einem Polymer, gebildet aus einem oder mehreren verschiedenen Monomeren der Formel (I) und einem polymeren Kondensationsprodukt aus Dicarbonsäuren gemäß Formel (IV) und Diaminen gemäß Formel (V), wobei die beiden Polymeren bevorzugt mit Epichlorhydrin vernetzt werden. Dabei bilden sich zwischen den beiden Polymeren überbrückende Einheiten aus, die sich vom Epichlorhydrin ableiten. In der Regel handelt es sich dabei um 2-Hydroxypropyleneinheiten ($-CH_2-CHOH-CH_2-$). Von den Monomeren gemäß Formel (I) sind Polyvinylmethylimidazol, Polyallylamin und Polymethallylamin besonders bevorzugt. Als Dicarbonsäuren werden geradkettige, gesättigte Dicarbonsäuren mit 2 bis 10 Kohlenstoffatomen, bevorzugt 5 oder 6 Kohlenstoffatome, eingesetzt und als Diamine oligomere Ethylendiamine mit 2 bis 5 Ethyleneinheiten bevorzugt zwei Ethyleneinheiten.

Zur Herstellung der bevorzugten Polybasen wird (oder werden) zunächst das (oder die) Monomer(e) gemäß Formel (I) in wäßriger Lösung polymerisiert. Nach beendeter Polymerisation wird das Kondensationsprodukt aus Dicarbonsäure (IV) und Diamin (V) als wäßrige Lösung zu dem Polymerisat gegeben und unter Hinzugabe von Epichlorhydrin vernetzt.

Das Kondensationsprodukt wird bevorzugt aus äquimolaren Anteilen von Dicarbonsäure (IV) und Diamin (V) hergestellt. Das Molverhältnis des Polymers, bestehend aus Monomereinheiten gemäß Formel (I), zu dem des Kondensationsproduktes beträgt 500-25:1, bevorzugt 50:1, besonders bevorzugt 25:1.

Die Konzentration des zugesetzten Epichlorhydrins beträgt 0,1 bis 0,5 Mol-% (bezogen auf das Polymer, bestehend aus Monomeren gemäß Formel (I)), bevorzugt 0,2 Mol-%, besonders bevorzugt 0,4 Mol-%.

Das Molekulargewicht der eingesetzten Polybasen beträgt 1000-200000 Dalton. Werden als Monomere gemäß Formel (I) Polyallylamin oder Polymethallylamin eingesetzt, so beträgt das Molekulargewicht bevorzugt 5000-100000 Dalton. Bei der Verwendung von Polyvinylmethylimidazol als Monomer gemäß Formel (I) beträgt das Molekulargewicht bevorzugt 5000-200000 Dalton.

Erfindungsgemäß können biologisch aktive Substanzen oder aktive Materialien, die in der Lage sind, biologisch aktive Substanzen zu produzieren, in die Polyelektrolytmembran eingeschlossen werden. Diese Membran gestattet den Transport von einer Vielzahl von Substanzen wie Nährstoffe, Substrat hin zu der biologisch aktiven Substanz und ist in der Lage, die dort produzierten Stoffe entweder selektiv zurückzuhalten oder nur solche passieren zu lassen (Semipermeabilität). Das biologisch aktive Material kann z.B. eine Zelle oder ein Zellmaterial oder ein chemischer oder biochemischer Reaktant sein. Als Zellen können z.B.

eingesetzt werden: Hybridomazellen oder genetisch modifizierte Zellen, hergestellt mittels rekombinanter DNA-Technologie, oder auch Lymphozytenzellen, die in der Lage sind, Antikörper oder Mikroorganismen für die Fermentation herzustellen.

Darüberhinaus können auch Mikroorganismen wie Bakterien enkapsuliert werden. Weiterhin ist es möglich, biologisch aktive Verbindungen wie Enzyme, Hormone, Antikörper oder Antibiotika einzukapseln, die kontrollierbar durch die Membran freigesetzt werden können oder - z.B. für katalytische Reaktionen - von der Membran zurückgehalten werden.

Die Permeabilität der Membran kann

a) über die Konzentrationen der eingesetzten Polysäuren und Polybasen

b) über den pH-Wert der wäßrigen Lösung der Polysäuren bzw. -basen

c) über das Molekulargewicht der eingesetzten Polysäuren und Polybasen sowie über die Verteilung des Molekulargewichts und

d) über die geeignete Auswahl, insbesondere der Polybasen und deren Ladungsdichte

gesteuert werden.

So führt beispielsweise eine Erhöhung der Polymerkonzentration gewöhnlich zu einer Erniedrigung der Permeabilität; andererseits führt z.B. ein Anteil der Monomeren mit positiver Ladung unter 30 Mol-% zu unzureichender Kapselstabilität.

Im folgenden wird die Erfindung anhand von Beispielen näher erläutert.

**Beispiel 1**

In einem 2 1-Glaskolben werden 294,9 g (3,15 mol) Allylamin-Hydrochlorid zusammen mit 105 ml Wasser gelöst. Dabei wird ein pH-Wert von 0 gemessen. Nun wird mit 4,23 g 5 %iger Ammoniaklösung auf einen pH-Wert von 4,1 eingestellt. Anschließend wird eine Lösung von 3,3 g 2,2'-Azo-bis-(2-amidinopropan)-dihydrochlorid, gelöst in 15 ml Wasser, zugetropft und unter Einleiten von Stickstoff bei 50 ° C 16 Stunden polymerisiert. Danach wird nochmals 3,3 g 2,2'-Azo-bis-(2-amidinopropan)-dihydrochlorid in 15 ml Wasser zugetropft und nochmals 4 Stunden polymerisiert. Zu dieser Polymerisatlösung werden nun 288,18 g des Kondensationsproduktes aus 1 Mol Adipinsäure und 1 Mol Diethylentriamin in Form einer.50 %igen wäßrigen Lösung zugefügt.

Anschließend werden 5,829 g (0,06 Mol) Epichlorhydrin als 5 %ige ethanolische Lösung zugefügt und 30 Minuten bei 50 ° C vernetzt. Nach dieser Zeit werden nochmals 5,829 g (0,06 Mol) Epichlorhydrin als 5 %ige ethanolische Lösung hinzugefügt und weitere 30 Minuten bei 50 ° C vernetzt. Nun wird mit Wasser auf eine Endkonzentration des Polymerisats von 40 % eingestellt.

Die Reaktion wurde durch Messung des K-Wertes in 1 %iger Lösung verfolgt:

K-Wert (vor der Polymerisation) $7{,}6 \bullet 10^3$

K-Wert (nach der Polymerisation) $9{,}5 \bullet 10^3$

K-Wert (nach der Vernetzung) $18{,}3 \bullet 10^3$

**Beispiel 2**

Analog Beispiel 1 wird ein Produkt aus 294,9 g (3,15 Mol) Allylamin-hydrochlorid und 323,75 g (1,25 Mol) des Kondensationsproduktes aus 1 Mol Korksäure und 1 Mol Diethylentriamin nach anschließender Vernetzung mit Epichlorhydrin in Form einer 50%igen wäßrigen Lösung erhalten.

**Beispiel 3**

In einem 1 l-Glaskolben werden 149,8 g (1,59 Mol) Allylamin-hydrochlorid vorgelegt und in 54,4 g Wasser gelöst. Anschließend wird mit 7,78 g 5 %iger Ammoniaklösung auf den pH-Wert 4,1 eingestellt. Nun werden 1,7 g 2,2′-Azo-bis-(2-amidinopropan)-dihydrochlorid, gelöst in 8 ml Wasser, zugesetzt und die Reaktionslösung unter Einleiten von Stickstoff auf 50 ° C erwärmt und 16 Stunden polymerisiert. Anschließend werden nochmals 1,7 g 2,2′-Azo-bis-(2-amidinopropan)-dihydrochlorid, gelöst in 8 ml Wasser, zugefügt und bei 50 bis 60 ° C weitere 4 Stunden polymerisiert.

Aus dieser wäßrigen Polymerisatlösung werden 144,76 g entnommen und 457,4 g des Kondensationsproduktes aus 1 Mol Adipinsäure und 1 Mol Diethylentriamin als 50%ige wäßrige Lösung zugesetzt. Nach 60-minütiger Reaktionszeit bei 50 bis 60 ° C wird durch Zutropfen von 37 g 5 %iger ethanolischer Lösung von Epichlorhydrin 30 Minuten bei 50 ° C vernetzt. Nach nochmaliger Vernetzung mit nochmals 37 g 5 %iger ethanolischer Lösung von Epichlorhydrin wird der pH-Wert der Reaktionslösung mit 70,7 g konzentrierter Salzsäure auf einen pH-Wert von 7,5 und anschließend mit 30,4 g Wasser auf eine Gesamtkonzentration

von 40 % eingestellt.
Der Verlauf der Polymerisation wurde anhand der Messung des K-Wertes verfolgt:
K-Wert (vor der Polymerisation) 8,6●$10^3$
K-Wert (nach der Polymerisation) 9,9●$10^3$
K-Wert (nach der Vernetzung) 21,6●$10^3$

**Beispiel 4**

Analog Beispiel 2 werden 149,8 g (1,59 Mol) Allylaminhydrochlorid polymerisiert und mit 178,8 g des Kondensationsproduktes aus 1 Mol Sebacinsäure und 1 Mol Diethylentriamin unter Hinzufügung von Epichlorhydrin vernetzt. Es wird eine 40%ige wäßrige Lösung erhalten.

**Beispiel 5**

In einem 2 l-Glaskolben werden 294,9 g (3,15 Mol) Allylamin-hydrochlorid in 105 ml Wasser eingetragen und mit 10,6 g 5 %iger Ammoniaklösung auf einen pH-Wert von 4,1 eingestellt. Nun fügt man 3,3 g 2,2'-Azo-bis-(2-amidinopropan)-dihydrochlorid, gelöst in 15 ml Wasser, hinzu und erwärmt unter Einleiten von Stickstoff auf 50 °C. Bei dieser Temperatur wird 16 Stunden polymerisiert. Anschließend werden nochmals 3,3 g 2,2'-Azo-bis-(2-amidinopropan)-dihydrochlorid, gelöst in 15 ml Wasser, hinzugegeben und weitere 4 Stunden bei 50 °C polymerisiert.
Zu dieser Polymerisationslösung werden nun 341,4 g (0,623 Mol) eines Kondensationsproduktes aus 1 Mol Adipinsäure und 1 Mol Triethylentetramin in Form einer 50%igen wäßrigen Lösung zugesetzt und nach 60-minütiger Reaktionszeit bei 50 bis 60 °C wird durch Zutropfen von 116,58 g einer 5 %igen ethanolischen Epichlorhydrinlösung 30 Minuten bei 50 °C vernetzt. Nach weiteren 30 Minuten werden nochmals 116,58 g 5 %ige ethanolische Epichlorhydrinlösung hinzugesetzt und abermals 30 Minuten vernetzt. Danach wird die Probe auf einen Gehalt an Polymerisat von 40 % eingestellt.
Die Reaktion wurde durch Messung des K-Wertes in 1%iger wäßriger Lösung verfolgt:
K-Wert (vor der Polymerisation) 8,1●$10^3$
K-Wert (nach der Polymerisation) 9,8●$10^3$
K-Wert (nach der Vernetzung) 22●$10^3$

**Beispiel 6**

Analog Beispiel 5 werden 294,9 g (3,15 Mol) Allylaminhydrochlorid polymerisiert und mit 161,35 g (0,623 Mol) eines Kondensationsproduktes aus 1 Mol Korksäure und 1 Mol Diethylentriamin unter Hinzugabe von Epichlorhydrin vernetzt. Danach wird die Probe auf einen Gehalt an Polymerisat von 40 % eingestellt.

**Beispiel 7**

In einem 2 l-Glaskolben werden 74,9 g (0,8 Mol) Allylamin-hydrochlorid, gelöst in 27,2 ml Wasser, vorgelegt und anschließend mit 7,78 g 5 %iger Ammoniaklösung auf einen pH-Wert von 4,1 eingestellt. Nun werden 0,85 g 2,2'-Azo-bis-(2-amidinopropan)-dihydrochlorid, gelöst in 8 ml Wasser, hinzugegeben und 16 Stunden unter Einleiten von Stickstoff bei 55 °C polymerisiert. Anschließend werden nochmals 0,85 g 2,2'-Azo-bis-(2-amidinopropan)-dihydrochlorid zugesetzt und weitere 3 Stunden bei 55 °C polymerisiert. Nun werden 457,43 g einer 50,5 %igen wäßrigen Lösung eines Kondensationsproduktes aus 1 Mol Adipinsäure und 1 Mol Diethylentriamin hinzugegeben und nach 60-minütiger Reaktionszeit bei 50-55 °C werden 37 g einer 5 %igen ethanolischen Lösung von Epichlorhydrin hinzugegeben und 20 Minuten vernetzt. Anschließend fügt man nochmals 37 g einer 5 %igen ethanolischen Lösung von Epichlorhydrin zu und vernetzt nochmals 30 Minuten. Anschließend wird mit 70,7 g konzentrierter Salzsäure auf einen pH-Wert von 7,5 und mit 30,4 ml Wasser auf einen Substanzgehalt von 40 % eingestellt.
Die Reaktion wurde anhand der in 1%iger wäßriger Lösung gemessenem K-Wert verfolgt:
K-Wert (vor der Polymerisation) 8.0●$10^3$
K-Wert (nach der Polymerisation) 20,8●$10^3$
K-Wert (nach der Vernetzung) 34,7●$10^3$

**Beispiel 8**

In einem 1 Liter Glaskolben werden 294,9 g (3,15 Mol) Allylamin-hydrochlorid in 105 ml Wasser gelöst und unter Kühlung 126,39 g (1,276 Mol) N-Vinyl-N-methylacetamid zugesetzt. Dann wird mit 10,5 g (0,154 Mol) konzentriertem Ammoniak auf pH = 4 eingestellt. Nun fügt man 10,53 g 2,2′-Azobis-(2-amidinopropan)hydrochlorid, gelöst in 48 g Wasser, zu. Unter Einleiten von Stickstoff wird auf 50 ° C Innentemperatur erhitzt und 16 Stunden auspolymerisiert. Dabei wurde nach 4 Stunden nochmals 10,53 g 2,2′-Azobis-(2-amidinopropan)hydrochlorid gelöst in 48 g Wasser hinzugegeben und der pH-Wert mit konzentriertem Ammoniak wieder auf pH = 4 eingestellt.
Es werden 660,38 g einer 63,3 %igen Lösung an Copolymerisat erhalten.

**Beispiel 9**

In einem 1 Liter Glaskolben werden 294,9 (3,15 Mol) Allylamin-hydrochlorid in 105 ml Wasser gelöst und unter Kühlung 126,39 g (1,345 Mol) N-Vinylimidazol zugefügt. Anschließend werden 10,53 g (2,2′-Azobis-(2-Amidinopropan)hydrochlorid, gelöst in 48 g Wasser, zugegeben. Dann wird unter Einleiten von Stickstoff auf 50 ° C erhitzt und 16 Stunden bei dieser Temperatur polymerisiert. Nach 4-stündiger Polymerisation wurden nochmals 10,53 g 2,2′-Azobis(2-amidinopropan)hydrochlorid, gelöst in 48 g Wasser zugegeben. Es werden 645 g einer 64,3 %igen wäßrigen Copolymerisatlösung erhalten.

**Beispiel 10**

In einem 4 Liter Glaskolben werden 1443 g (10 Mol) 1-Vinyl-3-methylimidazoliumchlorid und 56 g (0,5 Mol) 1-Vinyl-2-pyrrolidon in 3,8 l Wasser gelöst, das 38 g Kaliumperoxodisulfat als Initiator enthält. Der Ansatz wird 6 h bei 60 ° C unter Stickstoff polymerisiert. Man erhält eine klare gelb-braune 40 %ige Lösung mit neutralem pH-Wert. Der K-Wert beträgt 60.

**Beispiel 11**

Herstellung von Membrankapseln / Einschluß von Zellen

Eine Suspension von Hybridomazellen wird mit einer 4 Gew.-%igen λ-Carrageanlösung (Hersteller Sigma Chemie GmbH, München) in Dulbecco's Medium 1:1 (Massenverhältnis) verdünnt. Die Lösung wird über eine Düse getropft. Die Düse besteht aus einer Kanüle, Innendurchmesser 0,2 mm, Außendurchmesser 0,4 mm. Sie ist konzentrisch in einen Hohlzylinder eingelassen, so daß über den entstehenden Ringspalt ein tangentialer Luftstrom erzeugt werden kann, der die aus der Kanüle austretenden Tropfen abdrückt. Je nach Luftgeschwindigkeit betragen die Tropfengrößen 100 $\mu$m - 3000 $\mu$m. Die Tropfen fallen in eine Lösung der Polybase. Verwendet wird ein 0,5 Gew.-%ige Lösung der nach Beispiel 1 hergestellten Base. Es komplexiert sofort eine Polyelektrolytkomplexmembran aus. Die Kapseln werden mehrfach in einer Pufferlösung gewaschen und danach in ein Kulturmedium überführt und im Brutschrank gelagert.

**Beispiel 12**

Analog zu Beispiel 11 werden Tropfen einer Zellsuspension hergestellt. Sie fallen in eine Polybase. Verwendet wird eine 2 Gew.-%ige Lösung der Polybase, die nach Beispiel 10 hergestellt wurde. Die Kapseln werden analog Beispiel 11 weiter behandelt.

**Beispiel 13**

Die nach Beispiel 12 hergestellten Kapseln werden im Brutschrank kultiviert. Die Zellpopulationen wachsen an und nach 20 Tagen sind die Kapseln vollständig mit Zellen ausgefüllt. Die Zellen produzieren ca. 2 $\mu$g Antikörper pro Kapsel, d.h. ca. 1,1 mg Antikörper pro ml Reaktorvolumen.

**Beispiel 14**

Die nach Beispiel 11 hergestellten Kapseln werden im Brutschrank kultiviert. Die Kapseln lassen die produzierten Antikörper passieren. Nach 18 Tagen wurden ca. 0,7 mg Antikörper pro ml Kulturmedium abgegeben.

**Beispiel 15**

1 ml einer Antikörperlösung (0,091 mg IgG Faktor VIII/Type 1, Hersteller Calbiochem GmbH) in Phosphatpuffer pH 7,4 (0,00205 Mol $Na_2HPO_4$, 0,0045 Mol $NaH_2PO_4$) wird mit 1 ml einer 4 Gew.-%igen λ-Carragean-Lösung (Hersteller Sigma Chemie GmbH, München) gemischt.
Die Lösung wird aus einer Einwegspritze (Kanülendurchmesser 0,4 mm) in eine 100 ml Vorlage einer 0,5 Gew.-%igen Lösung der Polybase gemäß Beispiel 1 getropft. Nach 3 Minuten wurden die Mikrokapseln durch Dekantieren von überstehender Lösung der Polybase befreit, dreimal mit Phosphatpuffer pH 7,4 nachgewaschen und in 10 ml des Puffers suspendiert.

Analog werden Mikrokapseln mit der Polybase gemäß Beispiel 10 hergestellt. Die Vorlage der Polybase ist jedoch 2 Gew.-%ig. Nach 20 Minuten werden die Mikrokapseln durch Dekantieren von überstehender Lösung der Polybase befreit, einmal mit Phosphatpuffer pH 7,4 nachgewaschen und in 10 ml des Puffers suspendiert.

Nach definierten Zeiten (s. Tabelle) wird eine 1 ml Probe der beiden Suspensionen genommen und ihr Antikörpergehalt (Gew.-%) im Enzymimmunoassay (ELISA [Enzyme linked immunosorbent assay], Behringwerke AG, Marburg) bestimmt. Die Freigabe der gesamten Antikörpermenge aus den Mikrokapseln wird als 100 % angenommen:

| | 10 Minuten | 20 Stunden | 10 Tage |
|---|---|---|---|
| Polybase n. Bsp. 1 | < 0,1 % | 100 % | 100 % |
| Polybase n.Bsp. 10 | < 0,1 % | < 0,1 % | < 0,1 % |

**Patentansprüche**

**1.** Polyelektrolytmembrankapseln, bestehend aus einer semipermeablen Membran und einem von ihr einegeschlossenen biologisch aktiven Material, wobei die Membran aus einer biokompatiblen, nicht toxischen Polysäure und einer Polybase besteht, dadurch gekennzeichnet, daß die Polybase aus einem Polymer, gebildet aus wiederkehrenden Monomereinheiten der Formel (I)

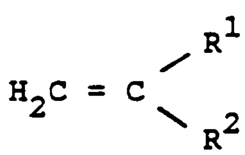

(I)

wobei
$R^1$ Wasserstoff oder Methyl und
$R^2$ eine Aminomethylgruppe, ein Imidazolrest oder ein Rest der Formel (II) ist

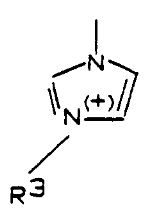

(II)

wobei
$R^3$ Wasserstoff, Methyl oder Ethyl bedeutet,
wobei miteinander verknüpfte Monomereinheiten auch voneinander verschiedene Reste $R^1$ und/oder $R^2$ enthalten können,
und
gegebenenfalls noch weiteren hydrophilen, biokompatiblen, keine elektrische Ladung aufweisenden Monomereinheiten

und
gegebenenfalls noch weiteren wasserlöslichen, biokompatiblen Polymeren, die gegebenenfalls mit dem Polymer, gebildet aus Monomereinheiten der Formel I und gegebenenfalls weiteren hydrophilen, biokompatiblen, keine elektrische Ladung aufweisenden Monomereinheiten, über überbrückende Einheiten vernetzt sind, besteht.

**2.** Polyelektrolytmembrankapseln, nach Anspruch 1, dadurch gekennzeichnet, daß die Polybase aus einem Polymer, gebildet aus wiederkehrenden Monomereinheiten der Formel (I) gemäß Anspruch 1 und noch weiteren, wasserlöslichen, biokompatiblen Polymeren, die gegebenenfalls mit dem Polymer, gebildet aus Monomereinheiten der Formel I, über überbrückende Einheiter vernetzt sind, besteht.

**3.** Polyelektrolytmembrankapseln nach Anspruch 1, dadurch gekennzeichnet, daß die Polybase aus einem Polymer, gebildet aus wiederkehrenden Monomereinheiten der Formel (I) gemäß Anspruch 1 und noch weiteren hydrophilen, biokompatiblen, keine elektrische Ladung aufweisenden Monomereinheiten besteht.

**4.** Polyelektrolytmembrankapseln nach Anspruch 1, dadurch gekennzeichnet, daß die Polybase aus einem Polymer, gebildet aus wiederkehrenden Monomereinheiten der Formel (I) gemäß Anspruch 1 und noch weiteren hydrophilen, biokompatiblen, keine elektrische Ladung aufweisenden Monomereinheiten und noch weiteren wasserlöslichen, biokompatiblen Polymeren, die gegebenenfalls mit dem Polymer, gebildet aus Monomereinheiten der Formel I und gegebenenfalls weiteren hydrophilen, biokompatiblen, keine elektrische Ladung aufweisenden Monomereinheiten, über überbrückende Einheiten vernetzt sind, besteht.

**5.** Polyelektrolytmembrankapseln nach einem oder mehreren der Ansprüche 1-4, dadurch gekennzeichnet, daß als weitere wasserlösliche, biokompatible Polymere ein Kondensationsprodukt aus Dicarbonsäuren der Formel (IV)

$HOOC - (CH_2)_n - COOH$ (IV)

wobei n 0 bis 8 ist,
und Aminen der Formel (V)

$H_2N - CH_2-(CH_2-NH-CH_2)_m-CH_2 - NH_2$ (V)

wobei m 1 bis 4 ist,
eingesetzt wird.

**6.** Polyelektrolytmembrankapseln nach Anspruch 5, dadurch gekennzeichnet, daß das Polymer, gebildet aus wiederkehrenden Monomereinheiten der Formel (I) gemäß Anspruch 1 und das Kondensationsprodukt aus den Verbindungen der Formel (IV) und (V) gemäß Anspruch 5 mit überbrückenden Einheiten vernetzt ist, die sich vom Epichlorhydrin ableiten.

**7.** Polyelektrolytmembrankapseln nach einem oder mehreren der Ansprüche 1-4, dadurch gekennzeichnet, daß als weitere hydrophile, biokompatible, keine elektrische Ladung aufweisenden Monomereinheiten N-Vinylpyrrolidon und/oder N-Vinylmethylacetamid und/oder Vinylcaprolactam eingesetzt werden.

**8.** Polyelektrolytmembrankapseln nach einem oder mehreren der Ansprüche 1-7, dadurch gekennzeichnet, daß der molare Anteil der hydrophilen, biokompatiblen, keine elektrische Ladung tragenden Monomereinheiten - bezogen auf das Gesamtmolekulargewicht der Polybase - 0-70 % beträgt.

**9.** Polyelektrolytmembrankapseln nach einem oder mehreren der Ansprüche 1-6, dadurch gekennzeichnet, daß der molare Anteil der wasserlösliche, biokompatiblen Polymere - bezogen auf das Gesamtmolekulargewicht der Polybase - 0-50% beträgt.

**10.** Polyelektrolytmembrankapseln nach Anspruch 1, dadurch gekennzeichnet, daß als Polybase ein Polymer, gebildet aus Methallylamin- oder Allylamin-Monomeren eingesetzt wird.

**11.** Polyelektrolytmembrankapseln nach Anspruch 10, dadurch gekennzeichnet, daß die Polybase ein Molekulargewicht von 5000-100000 Dalton besitzt.

**12.** Polyelektrolytmembrankapseln nach Anspruch 1, dadurch gekennzeichnet, daß als Polybase ein Polymer, gebildet aus Vinylmethylimidazol-Monomeren der Formel (III)

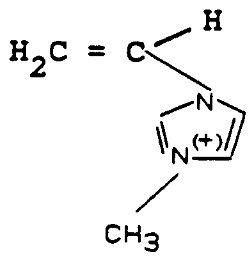

(III)

eingesetzt wird.

**13.** Polyelektrolytmembrankapseln nach Anspruch 12, dadurch gekennzeichnet, daß die Polybase ein Molekulargewicht von 5000-200000 Dalton besitzt.

**14.** Polyelektrolytmembrankapseln nach Anspruch 1, dadurch gekennzeichnet, daß als biologisch aktives Material Enzyme, Zellen oder Zellmaterial eingesetzt wird.

**15.** Verfahren zur Herstellung von Polyelektrolytmembrankapseln gemäß Anspruch 1, wobei man eine Suspension oder Lösung von aktivem Material in einer wäßrigen Lösung einer wasserlöslichen, nicht toxischen, biokompatiblen Polysäure herstellt und diese wäßrige Suspension oder Lösung in Tropfenform in eine wäßrige Lösung einer wasserlöslichen Polymerbase einbringt, wobei die Polysäure und die Polybase an der Phasengrenze miteinander reagiert und um das aktive Material eine wasserunlösliche, semipermeable Polymer-Polymer-Komplexmembran ausbildet, dadurch gekennzeichnet, daß die Polybase ein Polymer, bestehend aus wiederkehrenden Monomereinheiten der Formel (I) gemäß Anspruch 1, wobei miteinander verknüpfte Monomereinheiten auch voneinander verschiedene Reste $R^1$ und/oder $R^2$, wie in Anspruch 1 definiert, enthalten können und gegebenenfalls weiteren hydrophilen, biokompatiblen, keine elektrische Ladung aufweisende Monomereinheiten, ist und gegebenenfalls noch weitere, wasserlösliche, biokompatible Polymere, die gegebenenfalls mit dem Polymer, gebildet aus Monomereinheiten der Formel I und gegebenenfalls weiteren hydrophilen, biokompatiblen, keine elektrische Ladung aufweisenden Monomereinheiten, über überbrückende Einheiten vernetzt sind, enthält.

**16.** Verwendung der Polyelektrolytmembrankapseln gemäß Anspruch 14 zur Herstellung von Produkten durch Zellkulturen oder Enzyme.

**17.** Verwendung gemäß Anspruch 16, dadurch gekennzeichnet, daß die Produkte in Polyelektrolytmembrankapseln gebildet werden und diese nicht verlassen.

**18.** Verwendung gemäß Anspruch 16, dadurch gekennzeichnet, daß die Produkte in Polyelektrolytmembrankapseln gebildet werden und diese verlassen können.

## Claims

**1.** Polyelectrolyte membrane capsules composed of a semi-permeable membrane and of a biologically active material enclosed by it, the membrane being composed of a biocompatible, non-toxic polyacid and a polybase, wherein the polybase is composed of a polymer formed of repeating monomer units of the formula (I)

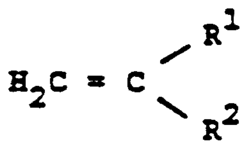

(I)

in which

$R^1$ is hydrogen or methyl, and

$R^2$ is an aminomethyl group, an imidazolyl radical or a radical of the formula (II)

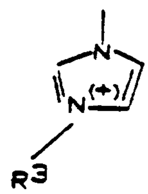

(II)

in which

$R^3$ denotes hydrogen, methyl or ethyl, it being possible for monomer units which are linked together also to contain radicals $R^1$ and/or $R^2$ which are different from one another, and, where appropriate, further hydrophilic, biocompatible monomer units which do not have an electric charge,

and, where appropriate, further water-soluble, biocompatible polymers which, where appropriate, are crosslinked via bridging units with the polymer formed of monomer units of the formula I and, where appropriate, with further hydrophilic, biocompatible monomer units which do not have an electric charge.

**2.** Polyelectrolyte membrane capsules as claimed in claim 1, wherein the polybase is composed of a polymer formed from repeating monomer units of the formula (I) as shown in claim I and further water-soluble, biocompatible polymers which are, where appropriate, crosslinked via bridging units with the polymer formed of monomer units of the formula I.

**3.** Polyelectrolyte membrane capsules as claimed in claim 1, wherein the polybase is composed of a polymer formed of repeating monomer units of the formula (I) as shown in claim 1 and further hydrophilic, biocompatible monomer units which have no electric charge.

**4.** Polyelectrolyte membrane capsules as claimed in claim 1, wherein the polybase is composed of a polymer formed of repeating monomer units of the formula (I) as shown in claim 1 and further hydrophilic, biocompatible monomer units which have no electric charge, and further water-soluble, biocompatible polymers which are, where appropriate, crosslinked via bridging units with the polymer formed of monomer units of the formula I and, where appropriate, with further hydrophilic, biocompatible monomer units which have no electric charge.

**5.** Polyelectrolyte membrane capsules as claimed in one or more of claims 1 - 4, wherein a condensation product of dicarboxylic acids of the formula (IV)

$HOOC - (CH_2)_n - COOH$ (IV)

in which n is 0 to 8, and amines of the formula (V)

$H_2N - CH_2-(CH_2-NH-CH_2)_m-CH_2 - NH_2$ (V)

in which m is 1 to 4, is used as further water-soluble, biocompatible polymers.

6. Polyelectrolyte membrane capsules as claimed in claim 5, wherein the polymer formed of repeating monomer units of the formula (I) as shown in claim 1 and the condensation products of the compounds of the formula (IV) and (V) as shown in claim 5 are crosslinked with bridging units derived from epichlorohydrin.

7. Polyelectrolyte membrane capsules as claimed in one or more of claims 1 - 4, wherein N-vinylpyrrolidone and/or N-vinyl-N-methylacetamide and/or vinylcaprolactam are used as further hydrophilic, biocompatible monomer units which have no electric charge.

8. Polyelectrolyte membrane capsules as claimed in one or more of claims 1 - 7, wherein the molar proportion of the hydrophilic, biocompatible monomer units which have no electric charge is 0 - 70 % based on the total molecular weight of the polybase.

9. Polyelectrolyte membrane capsules as claimed in one or more of claims 1 - 6, wherein the molar proportion of the water-soluble, biocompatible polymers is 0 - 50 % based on the total molecular weight of the polybase.

10. Polyelectrolyte membrane capsules as claimed in claim 1, wherein a polymer formed of methallylamine or allylamine monomers is used as polybase.

11. Polyelectrolyte membrane capsules as claimed in claim 10, wherein the polybase has a molecular weight of 5000 - 100,000 Dalton.

12. Polyelectrolyte membrane capsules as claimed in claim 1, wherein a polymer formed of vinylmethylimidazole monomers of the formula (III)

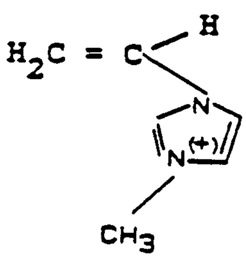

(III)

is used as polybase.

13. Polyelectrolyte membrane capsules as claimed in claim 12, wherein the polybase has a molecular weight of 5000 - 200,000 Dalton.

14. Polyelectrolyte membrane capsules as claimed in claim 1, wherein enzymes, cells or cell material is used as biologically active material.

15. A process for the preparation of polyelectrolyte membrane capsules as claimed in claim 1, in which a suspension or solution of active material in an aqueous solution of a water-soluble, non-toxic, biocompatible polyacid is prepared, and this aqueous suspension or solution is introduced in the form of drops into an aqueous solution of a water-soluble polymeric base, and in which the polyacid and the polybase react together at the phase boundary, and a water-insoluble, semipermeable polymer-polymer complex membrane is formed around the active material, which comprises the polybase being a polymer composed of repeating monomer units of the formula (I) as shown in claim 1, it being possible for monomer units which are linked together to contain radicals $R^1$ and/or $R^2$, as defined in claim 1, which are different from one another, and, where appropriate, further hydrophilic, biocompatible monomer units which have no electric charge, and, where appropriate, contains further water-soluble, biocompatible polymers which are, where appropriate, crosslinked via bridging units with the polymer formed of monomer units of the formula I and, where appropriate, with further hydrophilic, biocompatible monomer units which have no electric charge.

**16.** The use of the polyelectrolyte membrane capsules as claimed in claim 14 for the preparation of products by cell cultures or enzymes.

**17.** The use as claimed in claim 16, wherein the products are formed in polyelectrolyte membrane capsules and do not leave the latter.

**18.** The use as claimed in claim 16, wherein the products are formed in polyelectrolyte membrane capsules and are able to leave the latter.

**Revendications**

**1.** Capsules à membrane polyélectrolytique, comprenant une membrane semiperméable et une substance biologiquement active incorporée, la membrane étant constituée par un polyacide non toxique biocompatible et par une polybase, caractérisées en ce que la polybase se compose d'un polymère formé à partir de motifs monomères répétitifs de formule (I)

$H_2C=C(R^1R^2)$ (I)

dans laquelle
$R^1$ est un atome d'hydrogène ou un groupe méthyle, et
$R^2$ représente un groupe aminométhyle, un reste imidazole ou un este de formule (II)

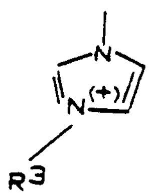

(II)

dans laquelle $R^3$ est hydrogène, méthyle ou éthyle,
les motifs monomères liés entre eux pouvant également contenir des restes $R^1$ et/ou $R^2$ différents les uns des autres,
et
éventuellement encore d'autres motifs monomères hydrophiles biocompatibles ne présentant pas de charge électrique,
et
éventuellement encore d'autres polymères biocompatibles hydrosolubles, qui sont éventuellement réticulés, par l'intermédiaire de motifs de pontage, avec le polymère formé à partir des motifs monomères de formule I et éventuellement d'autres motifs monomères hydrophiles biocompatibles ne présentant pas de charge électrique.

**2.** Capsules à membrane polyélectrolytique selon la revendication 1, caractérisées en ce que la polybase se compose d'un polymère formé à partir de motifs monomères répétitifs de formule (I) selon la revendication 1, et encore d'autres polymères biocompatibles hydrosolubles, qui sont éventuellement réticulés, par l'intermédiaire de motifs de pontage, avec le polymère formé à partir des motifs monomères de formule I.

**3.** Capsules à membrane polyélectrolytique selon la revendication 1, caractérisées en ce que la polybase se compose d'un polymère formé à partir de motifs monomères répétitifs de formule (I) selon la revendication 1, et encore d'autres motifs monomères hydrophiles biocompatibles ne présentant pas de charge électrique.

**4.** Capsules à membrane polyélectrolytique selon la revendication 1, caractérisées en ce que la polybase se compose d'un polymère formé à partir de motifs monomères répétitifs de formule (I) selon la revendication 1 et encore d'autres motifs monomères hydrophiles biocompatibles ne présentant pas de charge électrique et encore d'autres polymères biocompatibles hydrosolubles, qui sont éventuellement

réticulés, par l'intermédiaire de motifs de pontage, avec le polymère formé à partir des motifs monomères de formule I et éventuellement d'autres motifs monomères hydrophiles biocompatibles ne présentant pas de charge électrique.

5. Capsules à membrane polyélectrolytique selon une ou plusieurs des revendications 1 à 4, caractérisées en ce qu'on utilise, comme autres polymères hydrosolubles biocompatibles, des produits de condensation d'acides dicarboxyliques de formule (IV)

$HOOC\text{-}(CH_2)_n\text{-}COOH$ (IV)

dans laquelle n vaut 0 à 8,
et d'amines de formule (V)

$H_2N\text{-}CH_2\text{-}(CH_2\text{-}NH\text{-}CH_2)_m\text{-}CH_2\text{-}NH_2$ (V)

dans laquelle n vaut 1 à 4.

6. Capsules à membrane polyélectrolytique selon la revendication 5, caractérisées en ce que le polymère, formé à partir de motifs monomères répétitifs de formule (I) selon la revendication 1 et du produit de condensation des composés de formules (IV) et (V) selon la revendication 5, est réticulé avec des motifs de pontage qui dérivent de l'épichlorhydrine.

7. Capsules à membrane polyélectrolytique selon une ou plusieurs des revendications 1 à 4, caractérisées en ce qu'on utilise, comme autres motifs monomères hydrophiles biocompatibles ne présentant pas de charge électrique, la N-vinylpyrrolidone et/ou le N-vinylméthylacétamide et/ou le vinylcaprolactame.

8. Capsules à membrane polyélectrolytique selon une ou plusieurs des revendications 1 à 7, caractérisées en ce que la proportion molaire des motifs monomères hydrophiles biocompatibles ne portant pas de charge électrique est de 0 à 70 %, par rapport à la masse moléculaire totale de la polybase.

9. Capsules à membrane polyélectrolytique selon une ou plusieurs des revendications 1 à 6, caractérisées en ce que la proportion molaire des polymères hydrosolubles biocompatibles est de 0 à 50 %, par rapport à la masse moléculaire totale de la polybase.

10. Capsules à membrane polyélectrolytique selon la revendication 1, caractérisées en ce qu'on utilise, comme polybase, un polymère formé à partir des monomères méthallylamine et allylamine.

11. Capsules à membrane polyélectrolytique selon la revendication 10, caractérisées en ce que la polybase possède une masse moléculaire de 5000 à 100000 dalton.

12. Capsules à membrane polyélectrolytique selon la revendication 1, caractérisées en ce qu'on utilise, comme polybase, un polymère formé à partir des monomères vinylméthylimidazole de formule (III)

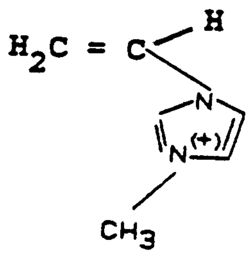

(III)

13. Capsules à membrane polyélectrolytique selon la revendication 12, caractérisées en ce que la polybase possède une masse moléculaire de 5000 à 200000 dalton.

**14.** Capsules à membrane polyélectrolytique selon la revendication 1, caractérisées en ce qu'on utilise, comme substance biologiquement active, des enzymes, des cellules ou de la substance cellulaire.

**15.** Procédé de fabrication de capsules à membrane polyélectrolytique selon la revendication 1, consistant à préparer une suspension ou une solution de substance active dans une solution aqueuse d'un polyacide biocompatible hydrosoluble non toxique, et à introduire goutte à goutte cette suspension ou solution aqueuse dans une solution aqueuse d'une base polymère hydrosoluble, le polyacide et la polybase réagissant entre eux à l'interphase et formant autour de la susbtance active une membrane insoluble dans l'eau, à base de complexe polymère polymère-semiperméable, caractérisé en ce que la polybase est un polymère se composant de motifs monomères répétitifs de formule (I) selon la revendication 1, les motifs monomères liés entre eux pouvant contenir des restes $R^1$ et/ou $R^2$ différents les uns des autes, tels que définis dans la revendication 1, et éventuellement encore d'autres motifs monomères hydrophiles biocompatibles ne présentant pas de charge électrique, et éventuellement encore d'autres polymères biocompatibles hydrosolubles, qui sont éventuellement réticulés, par l'intermédiaire de motifs de pontage, avec le polymère formé à partir des motifs monomères de formule I et éventuellement d'autres motifs monomères hydrophiles biocompatibles ne présentant pas de charge électrique.

**16.** Utilisation des capsules à membrane polyélectrolytique selon la revendication 14, pour la fabrication de produits, par cultures cellulaires ou par l'intermédiaire d'enzymes.

**17.** Utilisation selon la revendication 16, caractérisée en ce que ces produits sont formés dans des capsules à membrane polyélectrolytique qu'ils ne peuvent pas quitter.

**18.** Utilisation selon la revendication 16, caractérisée en ce que ces produits sont formés dans des capsules à membrane polyélectrolytique qu'ils peuvent quitter.